# EUROPEAN PATENT SPECIFICATION

(11) **EP 3 871 626 B1**
(45) Date of publication and mention of the grant of the patent: **17.04.2024**
(21) Application number: 19928295.5
(22) Date of filing: 30.12.2019
(51) Int. Cl.: A61B 18/12, A61B 18/14, A61B 18/00

(54) **EXPANDING NON-IMPLANTED ATRIAL SHUNT DEVICE**
EXPANDIERENDE NICHT-IMPLANTIERTE ATRIALE SHUNT-VORRICHTUNG
DISPOSITIF DE DÉRIVATION AURICULAIRE DILATABLE NON IMPLANTÉ

(43) Date of publication of application: 01.09.2021
(73) Proprietor: Shanghai Shape Memory Alloy Co., Ltd., Shanghai, 201612 (CN)
(72) Inventor: HE, Tao, Beijing 102200 (CN); DING, Yishou, Beijing 102200 (CN); ZHANG, Xibo, Beijing 102200 (CN); LI, Xiangyi, Beijing 102200 (CN); CAI, Jie, Beijing 102200 (CN); LIU, Liyun, Beijing 102200 (CN); HE, Ping, Beijing 102200 (CN); CHEN, Hao, Beijing 102200 (CN); ZHANG, Yuxin, Beijing 102200 (CN); PU, Zhongjie, Beijing 102200 (CN)
(74) Representative: J A Kemp LLP
(86) International application number: PCT/CN2019/129746
(87) International publication number: WO 2021/134154

(56) References cited:
- EP-B1- 3 698 840
- WO-A1-2019/085841
- WO-A2-2012/109557
- CN-A- 102 639 077
- CN-A- 102 940 524
- CN-A- 104 546 117
- CN-A- 108 135 653
- CN-A- 109 965 974
- CN-A- 110 062 606
- CN-A- 110 169 817
- US-A1- 2002 045 892
- US-A1- 2011 270 238

## Description

### TECHNICAL FIELD

The application relates to the technical field of medical equipment, in particular to a reaming type non-implanted atrial shunt device.

### BACKGROUND

Heart failure is a common and potentially fatal disease in humans. Although this disease has been best treated in hospitals, it is often difficult to be controlled and cured clinically. Especially, for disease "Heart failure with preserved ejection fraction (HFpEF)", the prevalence of this disease has increased significantly in the past few years, but the treatment of HFpEF is still a challenge for clinicians.

Heart failure with preserved ejection fraction is proposed as a concept of heart failure in relative to systolic insufficiency heart failure, which mainly refers to heart failure that occurs when the left ventricular diastolic function decreases and the systolic function is relatively normal. The disease is characterized by stiffness of the left ventricle, accompanied with decreased compliance and impaired relaxation, leading to increased end-diastolic blood pressure. Approximately one-third of heart failure patients suffer from "Heart failure with preserved ejection fraction".

Currently, an existing treatment method is to puncture an atrial septum of the heart to artificially create a hole to drain the blood pressure from a left atrium to a right atrium to reduce left atrial hypertension, thereby effectively improving "Heart failure with preserved ejection fraction".

CN 109965974 A discloses a transcatheter interventional atrial septostomy device.

### SUMMARY

Therefore, the present application provides a reaming type non-implanted atrial shunt device that can make the artificial hole difficult to be reclosed so as to drain blood pressure from the left atrium to the right atrium.

The invention provides a device according to claim 1. Embodiments of the invention are defined in the claims.

The technical solution of the present application has the following advantages:
1. The reaming type non-implanted atrial shunt device of the present application comprises a catheter component, a radio frequency component, disposed on an end of the catheter component extending into a human body, and a radio frequency ablation instrument, disposed on the other end of the catheter component and connected with the radio frequency component, wherein after the radio frequency component extends into a puncture hole, the radio frequency ablation instrument releases a radio frequency energy, and the radio frequency component ablates a wall of the puncture hole. The radio frequency component is delivered to the position of the puncture hole. When the radio frequency ablation instrument releases energy, the radio frequency component causes the hole wall tissue to generate heat and ablate the atrial septum at a target position to form a hole with fixed size, thereby achieving the purpose of creating a hole. Using radio frequency components to create a hole through radio frequency make the hole not easy to reclose. The hole can effectively drain the left atrium blood into the right atrium, reduce pressure of the left atrium, thereby achieving the purpose of treatment.
2. In the reaming type non-implanted atrial shunt device of the present application, the setting of a balloon enable the electrode to effectively ablate a wall of the puncture hole, making the creating of the hole convenient and accurate.
3. In the reaming type non-implanted atrial shunt device of the present application, a tip is provided to facilitate the radio frequency component to protrude from a sheath and move in the body, and to prevent damage to the body tissue.

### BRIEF DESCRIPTION OF THE DRAWINGS

In order to describe the specific embodiments of the present application or the technical solutions in the prior art more clearly, the drawings used in the specific embodiments or the description of the prior art will be introduced briefly. Obviously, the drawings represent some embodiments of the present application and other drawings can be obtained based on these drawings without paying inventive work for a person skilled in the art.
Figure 1 is a schematic structural view of a reaming type non-implanted atrial shunt device of the present application;
Figure 2 is a schematic structural view of the radio frequency component of the present application;
Figure 3 is a schematic structural view of an interior of the radio frequency component of the present application;
Figure 4 is a schematic structural view of a temperature sensor in the radio frequency component of the present application;
Figure 5 is a schematic structural view of a cross-sectional view perpendicular to a centerline of the balloon of the radio frequency component of the present application;
Figure 6 is a schematic structural view of a catheter component of the present application;
Figure 7 is a schematic structural view of a cross-sectional view of a catheter component of the present application perpendicular to an extending direction of the catheter component;
Figure 8 is a schematic structural view of the connection between the radio frequency component and the catheter component of the present application;
Figure 9 is a schematic structural view of an electrode according to Embodiment 1 of the present application;
Figure 10 is a schematic structural view of an electrode according to Embodiment 2 of the present application;
Figure 11 is a schematic structural view of an electrode according to Embodiment 3 of the present application;
Figure 12 is a schematic structural view of an electrode according to Embodiment 4 of the present application;
Figure 13 is a schematic structural view of an electrode according to Embodiment 5 of the present application;
Figure 14 is a schematic structural view of a balloon according to Embodiment 1 of the present application;
Figure 15 is a schematic structural view of a balloon according to Embodiment 2 of the present application;
Figure 16 is a schematic structural view of a balloon according to Embodiment 3 of the present application;
Figure 17 is a schematic structural view of a balloon according to Embodiment 4 of the present application;
Figure 18 is a schematic structural view of a radio frequency component of the present application in a retracted state or an unreleased state;
Figure 19 is a schematic structural view of a reaming type non-implanted atrial shunt device of the present application in use state.

### Reference signs:

1. radio frequency component; 11. balloon; 12. electrode; 13. tip; 121. electrode wire; 122. temperature wire; 123. temperature sensor; 2. catheter component; 21. outer tube; 22. multi-cavity inner tube; 23. guide filament channel; 221. wire channel; 222. pressure charging channel; 223. pressure through hole; 224. blocking structure; 225. wire through hole; 3. four-way valve; 31. electric connector; 32/53. Luer taper; 33. third valve opening; 4. radio frequency ablation instrument; 41. connection module; 42. control module; 43. power output module; 44. temperature control module; 412. anode; 5. sheath; 51. operation handle; 52. sheath parts; 54. catheter channel interface; 6. pressure pump; 61. control handle; 62. connecting catheter; 63. connecting joint; 7. guide filament.

### DETAILED DESCRIPTION

The present application will be further described in detail below with reference to the drawings and examples. Obviously, the described embodiments represent part of, but not all of the embodiments of the present application. In the description of the present application, it should be understood that the terms "center", "upper", "lower", "left", "right", "vertical", "horizontal", "inside" and "outside" indicate orientation or positional relationship which are based on the orientation or positional relationship shown in the drawings, and it is merely for convenience of describing the present application and simplifying the description, rather than indicating or implying that the indicated device or element must have a particular orientation, and be constructed and operated in a particular orientation. Therefore these terms should not be understood as limiting the scope of the present application. In addition, the terms "first", "second", and "third" are only used for descriptive purposes, and cannot be understood as indicating or implying relative importance.

In the description of the present application, unless otherwise explicitly stated or specified, it should be noted that the terms "mounted", "connected" and "connecting" should be understood in a broad sense. For example, a connection can be a fixed or detachable connection, or an integral connection; a mechanical connection or an electrical connection; a direct or indirect connection through an intermediate medium, and it can be an internal communication between two elements. For a person skilled in the art, the specific meaning of the above-mentioned term in the present application can be understood in specific situation.

In addition, the technical features involved in the different embodiments of the present application described below can be combined with each other as long as they do not conflict with each other.

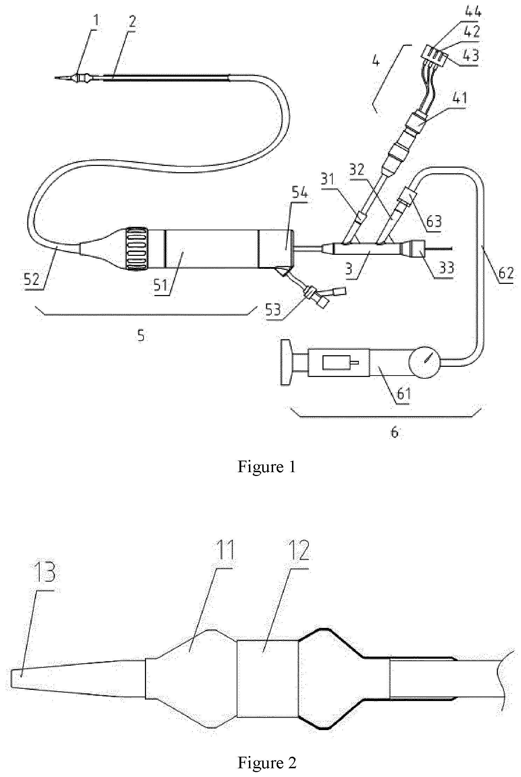

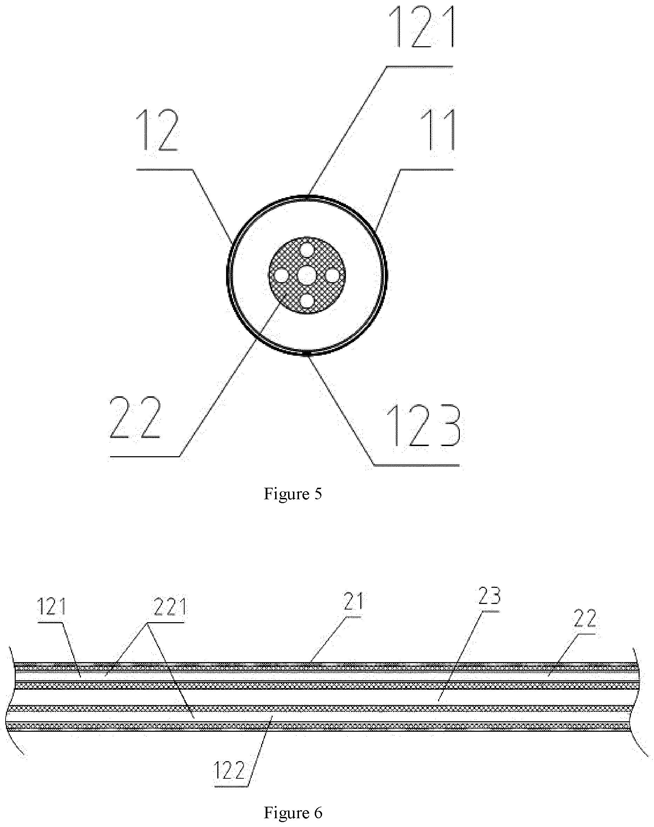

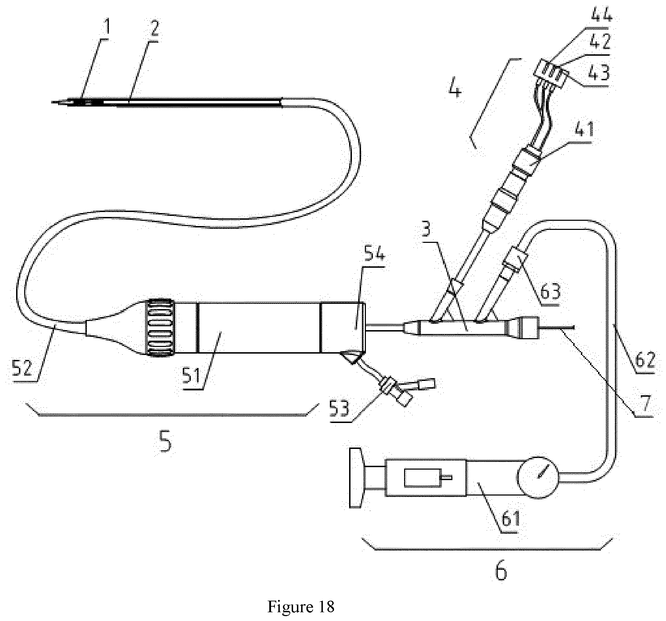

The reaming type non-implanted atrial shunt device in the embodiment of present application is used to create a hole in the tissue wall. In the embodiment, for example, the hole is created in the atrial septum of the heart, and the hole is not easy to be closed again. As shown in Figures 1 to 19, the reaming type non-implanted atrial shunt device comprises a radio frequency component 1, a catheter component 2, a four-way valve 3, a radio frequency ablation instrument 4, a sheath 5 and a pressure pump 6.

As shown in Figure 1, Figure 18 and Figure 19, the radio frequency component 1 is arranged on an end of the catheter component 2 that extends into human body, and the other end of the catheter component 2 is connected with a four-way valve 3, wherein a first valve opening is connected to the pressure pump 6 through a Luer taper 32 provided on the first valve, and a second valve opening is connected to the radio frequency ablation instrument 4 through an electrical connector 31 provided on the second valve, and the third valve opening 33 is used to insert a guide filament 7, and a fourth valve opening is connected a the catheter component 2. A sheath 5 is sleeved on external side of the catheter component 2, and the sheath 5 is arranged close to the four-way valve 3.

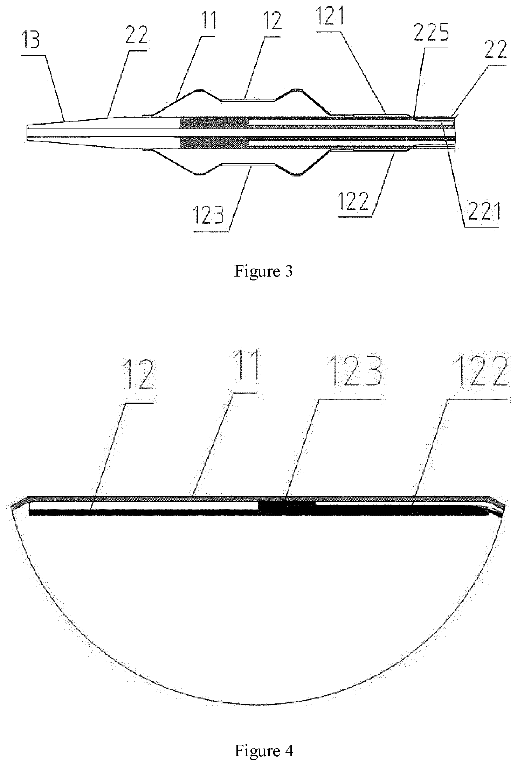

As shown in Figures 2 and 3, the radio frequency component 1 comprises a balloon 11 that expands after charging pressure and retracts after discharging pressure, and an electrode 12 fixedly arranged on an external side of the balloon 11. In order to facilitate use of the radio frequency component 1, a tip 13 is also arranged at a front end of the radio frequency component 1.

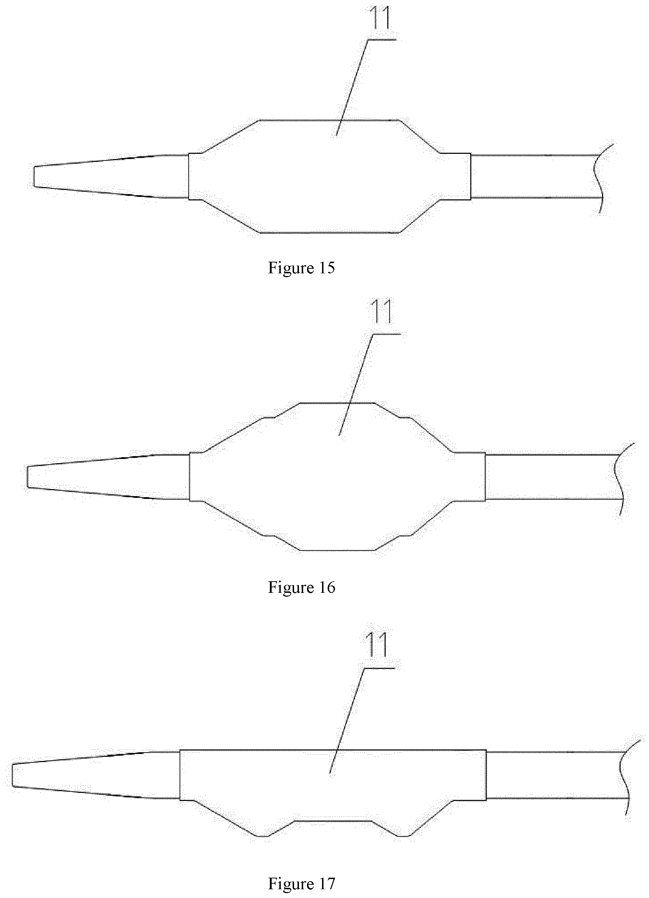

The balloon 11 and the multi-cavity inner tube 22 are fixedly connected by welding or bonding. The balloon 11 becomes larger after charging pressure, and two ends of the balloon 11 are smoothly connected with the multi-cavity inner tube 22. The balloon 11 can support the electrode 12, such that the electrode 12 can tightly contact a wall of the puncture hole. As shown in Figure 15, the balloon 11 is expanded to have a cylindrical shape, which is not limited thereto, as long as the balloon 11 expanded to have a shape that achieves the purpose of radiofrequency ablation by using the electrode 12.

As an alternative embodiment, as shown in Figure 16, a middle part of the balloon 11 after being expanded has a protrusion protruding outward along a diameter of the balloon 11, and the protrusion is arranged around the middle line of the balloon 11 or at a predetermined angle. A side wall of the protrusion away from the balloon 11 is parallel to the midline of the balloon 11, and the predetermined angle can be adjusted according to usage requirements, for example the protrusion surrounds a half circle of the middle line of the balloon 11, or a protrusion covering 60 degrees is arranged every 30 degrees around the balloon 11.

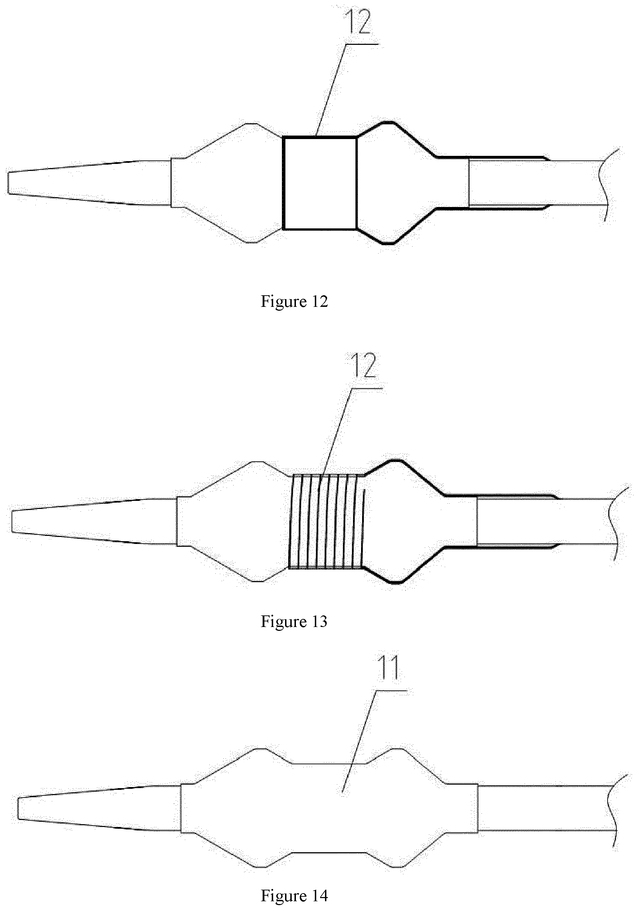

As an alternative embodiment, as shown in Figures 14 and 17, after expansion of the balloon 11, a middle part of the balloon comprises a groove recessed inward along a diameter of the balloon 11, and the electrode 12 is fixed in the groove, and the groove is arranged around the middle line of the balloon 11 or at a predetermined angle. The predetermined angle can be adjusted according to usage requirements, for example the protrusion surrounds a half circle of the middle line of the balloon 11, or a protrusion covering 60 degrees is arranged every 30 degrees around the balloon 11, or one side is flush with the middle line, and the other side is provided with a groove recessed inwardly in the middle part and the protrusion has a concave structure as whole.

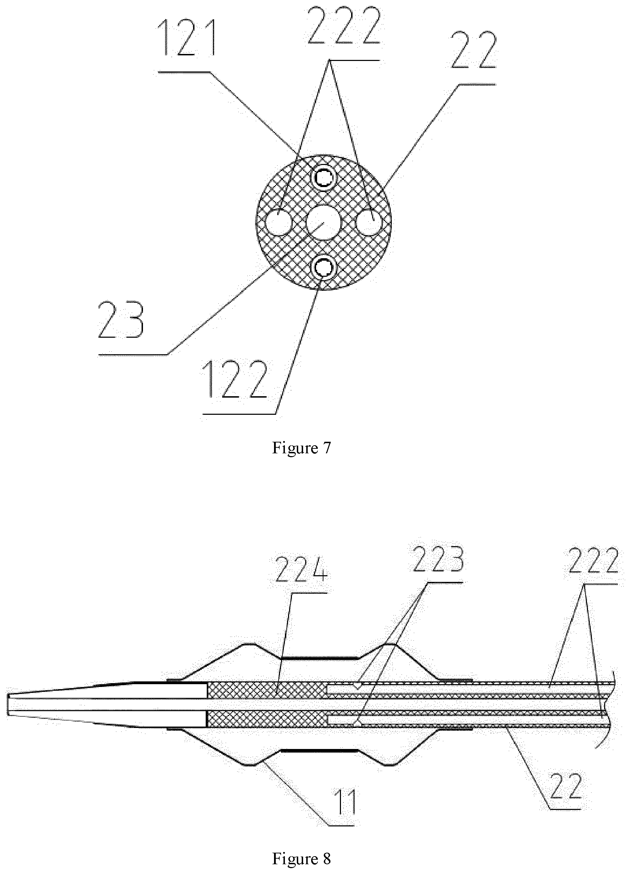

As shown in Figure 8, the balloon 11 is connected to a pressure pump 6 through a pressure charging channel 222 provided in the multi-cavity inner tube 22. The pressure pump 6 is a device for charging pressure to the balloon 11 and discharging pressure from the balloon 11, and a charging pressure material or discharging pressure material is liquid or gas, which is not limited thereto, as long as the material meets usage requirements.

As shown in Figure 1, the pressure pump 6 comprises a control handle 61, and a connecting pipe 62 with two ends respectively connected to the control handle 61 and a connecting joint 63, and the connecting joint 63 is connected to a Luer taper 32.

As shown in Figures 2-8, the electrode 12 is fixed on the balloon 11, and at least on the middle part of the balloon 11. The electrode 12 is connected to the radio frequency ablation instrument 4 through the electrode wire 121 disposed on the catheter component 2. In the embodiment, the electrode is winded around an external side of the balloon and has a shape selected from the group consisting of filament shape, net-like shape, lattice shape, or sheet shape, or spiral filament shape, which is not limited thereto, as long as the electrode 12 can be used to achieve the purpose of radiofrequency ablation. The electrode wire 121 is fixedly connected to the electrode 12 by welding or conductive adhesive bonding.

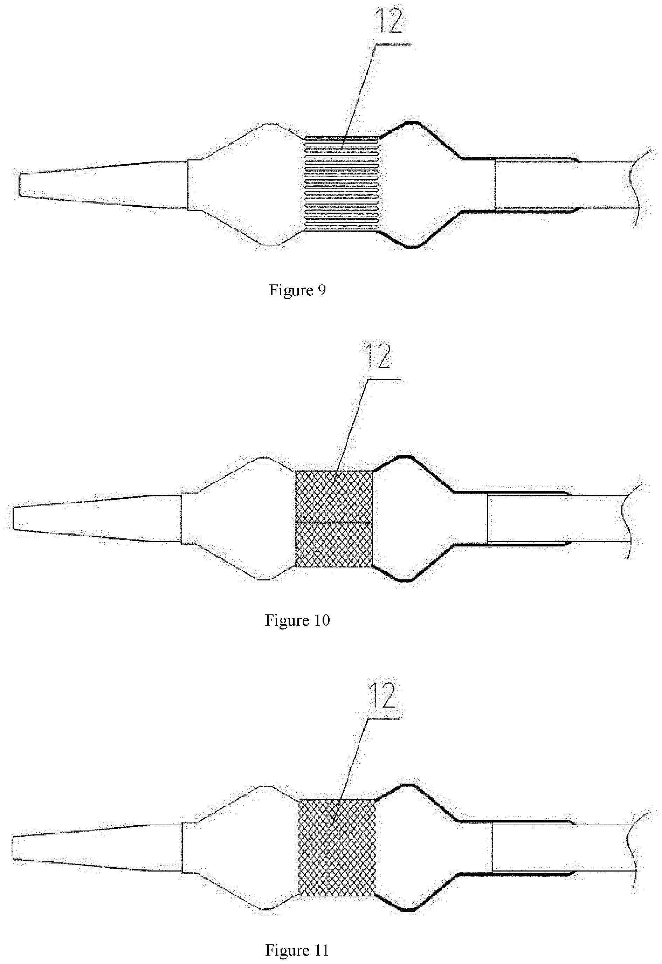

The embodiment of Figure 9 comprises a electrode 12 having a spiral filament shape, and a consolidation method comprises bonding, etching, printing, coating, etc.

The embodiment of Figure 10 comprises a mesh electrode 12, and a sheet-like conductive wire mesh is uniformly pasted on an outer surface of the balloon 11 along a circumferential direction.

The embodiment of Figure 11 comprises a lattice electrode 12 or a cutting stent electrode 12, and a consolidation method comprises bonding, etching, coating, etc.

The embodiment shown in Figure 12 comprises a sheet electrode 12, and a consolidation method comprises bonding, etching, coating, and the like.

The embodiment shown in Figure 13 comprises a spiral filament electrode 12, and a consolidation method comprises bonding, etching, coating, and the like.

As shown in Figure 4, in order to detect the temperature of the electrode 12, the radio frequency component 1 further comprises at least one temperature sensor 123 arranged between the balloon 11 and the electrode 12. The temperature sensor 123 is connected to the radio frequency ablation instrument 4 through at least one temperature wire 122 arranged in the catheter component 2. There may be one temperature sensor 123 or a plurality of temperature sensors 123 distributed along the circumference.

The radio frequency ablation instrument 4 comprises a connection module 41 connected to the electrical connector 31, a power output module 43 connected to the connection module 41, a temperature control module 44, and a control module 42. In the embodiment, the electrode 12 is a cathode, and an anode 412 of the radio frequency ablation instrument 4 is attached to human body. During operation of the radio frequency ablation instrument 4, the energy emitted by the radio frequency ablation instrument 4 flows through the electrode wire 121, the connection module 41, the electrical connector 31, the electrode wire 121, the electrode 12, the human body, the anode 412, and an extracorporeal electrode wire electrode to flows back to the radio frequency ablation device 4, thus forming a circuit loop.

The tip 13 is fixedly connected to the distal end of the multi-cavity inner tube 22 and the balloon 11, and the tip 13 has a tapered or spherical structure, the size of which less than or equal to an outer diameter of the catheter component 2, and the method of the fixed connection comprises welding or bonding.

The catheter component 2 comprises an outer tube 21, and a multi-cavity inner tube 22 arranged in the outer tube 21, the outer tube 21 is sleeved in the sheath 5, and the multi-cavity inner tube 22 comprises a plurality of separated channels, the plurality of channels comprise at least one wire channel 221 for setting a wire, at least one pressure charging 222 for inputting liquid or gas, and a guide filament channel 23 for passing the guide filament 7. As shown in Figure 7, two pressure charging channels 222, two wire channels 221, temperature wire 122 and the electrode wire 121 can be arranged in the same wire channel 221 or separately arranged.

As shown in Figure 3, one end of the pressure charging 222 that extends to the balloon 11 has a pressure through hole 223 communicated with the balloon 11, and the pressure charging 222 and the balloon 11 are connected as a sealed chamber. In the embodiment, a blocking structure 224 is arranged at the ends of the pressure charging 222 and the wire channel 221, and the blocking structure 224 seals the pressure charging 222 and the wire channel 221 to avoid leakage or inflow of liquid or gas.

The multi-cavity inner tube 22 is connected to the balloon 11, and at least one wire through hole 225 connecting wire channel 221 and an external side of the multi-cavity inner tube 22 is arranged at a position near the junction of the multi-cavity inner tube 22 and the balloon 11. The electrode wire 121 and the temperature wire 122 pass through the multi-cavity inner tube 22 from the wire through hole 225, and are respectively connected to the corresponding electrode 12 and the temperature sensor 123. A gap between the wire through hole 225 and the wire is filled with insulating glue to prevent liquid from entering the wire channel 221 to affect an insulation effect.

The sheath 5 comprises an operation handle 51, a sheath part 52, a Luer taper 53, and a catheter channel interface 54.

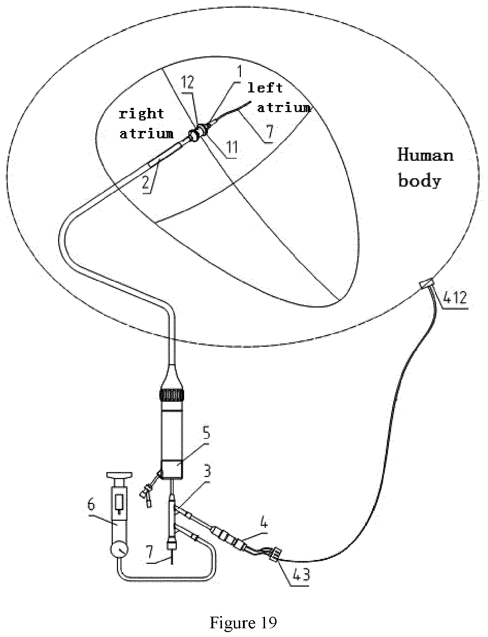

As shown in Figure 19, in the reaming type non-implanted atrial shunt device, the catheter component 2 is retracted into the sheath 5 or is in a unreleased state. At this time, the radio frequency component 1 is in a retracted state and retracted in the interior of the sheath 5, and during the operation, the radio frequency component 1 can be pushed out of the outlet of the sheath 5 or pulled back into the sheath 5.

After puncturing of the cardiac atrial septal is completed, the radio frequency component 1 is delivered into the right atrium through the guide filament 7, and the groove on the balloon 11 is aligned with the punctured hole in the atrial septal. The pressure pump 6 is controlled to charge pressure to the balloon 11, and the balloon 11 is expanded to make the electrode 12 fit with the inner surface of the puncture hole. A working pressure of the balloon 11 can be controlled by the pressure pump 6 to maintain the pressure within a suitable pressure range. The radio frequency ablation instrument 4 is controlled to start radio frequency energy output and a radio frequency current is transmitted to the electrode 12 through the electrical connector 31 and the electrode wire 121 and a high-temperature area is formed on the surface of the electrode 12 in contact with the atrial septal puncture hole, and the tissue is heated to achieve ablation to form a hole in the atrial septal.

During the working process, the temperature sensor 123 collects the temperature parameters in an ablation area, and transmits the temperature parameters to the radio frequency ablation instrument 4 through the temperature wire 122 and the electrical connector 31 to realize temperature feedback and control so as to realize a precise hole making operation through appropriate ablation time and temperature control.

After creating the hole is completed, the pressure pump 6 is controlled to vacuum the balloon 11 until the balloon 11 is completely retracted, and then the radio frequency component 1 is pulled back into the sheath 5 and pulled out to the outside of the body.

After creating a hole in heart atrial septal is completed, in the left atrium which initially works under high pressure, the blood will be shunted to the right atrium through the hole in the heart atrial septal as the blood pressure in the left atrium is higher than that in the right atrium, thereby reducing the pressure in the left atrium and achieving the purpose of shunting the blood. The radio frequency component 1 is used to create a hole through radio frequency, and the created hole is not easy to be closed again. The hole created can effectively drain the blood pressure of the left atrium into the right atrium to achieve the purpose of treatment.

Certainly, the above-mentioned embodiments are only examples for clear description, and are not intended to restrict the embodiment. The scope of the invention is defined by the appened claims.

## Claims

1. A device, comprising
a catheter component (2),
a radio frequency component (1), disposed on an end of the catheter component (2) extending into a human body, and
a radio frequency ablation instrument (4), disposed on the other end of the catheter component (2) and connected with the radio frequency component (1),
the radio frequency component (1) comprises a balloon (11) that expands by charging pressure or retracts by discharging pressure, and an electrode (12) fixedly disposed on the balloon (11); and
a charging and discharging pressure device is disposed on the other end of the catheter component (2) and communicating with the balloon (11), the electrode (12) is connected with the radio frequency ablation instrument (4) through an electrode wire (121);
wherein the balloon (11) is cylindrical after being expanded, or a middle part of the balloon (11) after being expanded has protrusions protruding outwardly, preferably, the protrusions are arranged around a circle of the midline of the balloon (11) or configured at a set angle, and a side wall of the protrusion away from the balloon (11) is parallel to the midline of the balloon (11);
the middle part of the balloon (11) after being expanded has a groove recessed inwardly, and the electrode (12) is fixed in the groove;
the groove is arranged around a circle of the midline of the balloon (11) or configured at a set angle,
the catheter component (2) comprises an outer tube (21), and a multi-cavity inner tube (22) disposed in the outer tube, and
the multi-cavity inner tube (22) has a plurality of separated channels therein, and the plurality of channels comprises,
at least one wire channel (221) for setting a wire,
at least a pressure charging channel (222) for inputting liquid or gas, and
a guide filament channel (23) for introduction of the guide filament (7), one end of the pressure charging channel (222) that extends to the balloon (11) is provided with a pressure through hole (223) which is communicating with the balloon (11) and the pressure charging (222) and the balloon (11) are connected to form a sealed chamber,
the multi-cavity inner tube (22) is connected to the balloon (11), and at least one wire through hole (225) is disposed close to a connection portion of the multi-cavity inner tube (22) and the balloon (11) and used to communicate the wire channel (221) and an external side of the multi-cavity inner tube (22), and
an electrode wire (121) and an at least one temperature wire (122) are respectively connected with the corresponding electrode (12) and an at least one temperature sensor (123) the radio frequency component (1) after passing through the multi-cavity inner tube (22) from the wire through hole (225),
the other end of the catheter component (2) is provided with a four-way valve (3), wherein
a first valve opening is configured to be connected to a pressure pump (6) through a Luer taper (32) provided thereon,
a second valve opening is connected to the radio frequency ablation instrument (4) through an electrical connector (31) provided thereon, and
a third valve opening (33) is configured to be used to pass the guide filament (7),
a blocking structure (224) is arranged at the ends of the pressure charging channel (222) and the wire channel (221), and the blocking structure (224) seals the pressure charging (222) and the wire channel (221) 1,
a gap between the wire through hole (225) and the wire is filled with insulating glue.

2. The device according to claim 1, wherein
the electrode (12) is winded around an external side of the balloon (11) and has a shape selected from the group consisting of filament shape or spiral filament shape.

3. The device according to any one of claims 1-2, wherein
the at least one temperature sensor (123) is disposed between the balloon (11) and the electrode (12), and
the temperature sensor (123) is connected with the radio frequency ablation instrument (4) through the at least one temperature wire (122) disposed in the catheter component (2).

4. The device according to any one of claims 1-3, wherein,
a tip (13) is further disposed at a front end of the radio frequency component (1), and the tip (13) has a conical or spherical structure having a diameter less than or equal to an outer diameter of the catheter component (2).

5. The device according to any one of claims 1-4, further comprising a sheath (5) sleeved on an external side of the catheter component (2), .

6. The device according to any one of claims 1-5, wherein
the electrode (12) is a cathode, and an anode (412) of the radio frequency ablation instrument (4) is configured to be attached to a human body.

## Patentansprüche

1. Vorrichtung, die umfasst:
eine Katheterkomponente (2),
eine Hochfrequenzkomponente (1), die an einem Ende der Katheterkomponente (2) angeordnet ist, das sich in einen menschlichen Körper erstreckt, und
ein Hochfrequenz-Ablationsinstrument (4), das an dem anderen Ende der Katheterkomponente (2) angeordnet und mit der Hochfrequenzkomponente (1) verbunden ist,
wobei die Hochfrequenzkomponente (1) einen Ballon (11) umfasst, der sich durch Ladedruck ausdehnt oder durch Entladedruck zusammenzieht, und eine Elektrode (12) umfasst, die fest an dem Ballon (11) angeordnet ist; und
wobei am anderen Ende der Katheterkomponente (2) eine Lade- und Entladedruckvorrichtung angeordnet ist, die mit dem Ballon (11) in Verbindung steht, wobei die Elektrode (12) über einen Elektrodendraht (121) mit dem Hochfrequenz-Ablationsinstrument (4) verbunden ist;
wobei
der Ballon (11) nach seiner Ausdehnung zylindrisch ist, oder ein mittlerer Teil des Ballons (11) nach seiner Ausdehnung nach außen vorstehende Vorsprünge aufweist, wobei die Vorsprünge vorzugsweise um einen Kreis der Mittellinie des Ballons (11) angeordnet oder in einem bestimmten Winkel konfiguriert sind und eine Seitenwand des Vorsprungs, die von dem Ballon (11) abgewandt ist, parallel zur Mittellinie des Ballons (11) verläuft;
wobei der mittlere Teil des Ballons (11) nach dem Ausdehnen eine nach innen vertiefte Rille aufweist und die Elektrode (12) in der Rille befestigt ist;
wobei die Rille um einen Kreis der Mittellinie des Ballons (11) angeordnet oder in einem bestimmten Winkel konfiguriert ist,
wobei die Katheterkomponente (2) eine Außenröhre (21) und eine in der Außenröhre angeordnete Mehrfach-Hohlraum-Innenröhre (22) umfasst, und
wobei die Mehrfach-Hohlraum-Innenröhre (22) eine Vielzahl von getrennten Kanälen darin aufweist und die Vielzahl von Kanälen Folgendes umfasst:
mindestens einen Drahtkanal (221) zum Einsetzen eines Drahtes,
mindestens einen Druckbeaufschlagungskanal (222) für die Zufuhr von Flüssigkeit oder Gas, und
einen Führungsfadenkanal (23) zum Einführen des Führungsfadens (7),
wobei ein Ende des Druckbeaufschlagungskanals (222), das sich zum Ballon (11) erstreckt, mit einem Druckdurchgangsloch (223) versehen ist, das mit dem Ballon (11) in Verbindung steht, und die Druckbeaufschlagung (222) und der Ballon (11) verbunden sind, um eine abgedichtete Kammer zu bilden,
wobei die Mehrfach-Hohlraum-Innenröhre (22) mit dem Ballon (11) verbunden ist, und mindestens ein Drahtdurchgangsloch (225) in der Nähe eines Verbindungsabschnitts der Mehrfach-Hohlraum-Innenröhre (22) und des Ballons (11) angeordnet ist und zur Verbindung des Drahtkanals (221) und einer Außenseite der Mehrfach-Hohlraum-Innenröhre (22) dient, und
wobei ein Elektrodendraht (121) und mindestens ein Temperaturdraht (122) jeweils mit der entsprechenden Elektrode (12) und mindestens einem Temperatursensor (123) der Hochfrequenzkomponente (1) verbunden sind, nachdem sie von dem Drahtdurchgangsloch (225) durch die Mehrfach-Hohlraum-Innenröhre (22) hindurchgeführt wurden,
wobei das andere Ende der Katheterkomponente (2) mit einem Vier-WegeVentil (3) versehen ist, wobei
eine erste Ventilöffnung so konfiguriert ist, dass sie über einen daran vorgesehenen Luer-Konus (32) mit einer Druckpumpe (6) verbunden werden kann,
eine zweite Ventilöffnung mit dem Hochfrequenz-Ablationsinstrument (4) über einen daran befindlichen elektrischen Anschluss (31) verbunden ist, und
eine dritte Ventilöffnung (33) so konfiguriert ist, dass sie zum Durchführen des Führungsfilaments (7) verwendet wird,
wobei eine Absperrstruktur (224) an den Enden des Druckbeaufschlagungskanals (222) und des Drahtkanals (221) angeordnet ist und die Absperrstruktur (224) die Druckladung (222) und den Drahtkanal (221) abdichtet,
wobei ein Spalt zwischen dem Drahtdurchgangsloch (225) und dem Draht mit Isolierkleber gefüllt ist.

2. Vorrichtung nach Anspruch 1, wobei
die Elektrode (12) um eine Außenseite des Ballons (11) gewickelt ist und eine Form aufweist, die aus der Gruppe ausgewählt ist, die aus der Form eines Fadens oder eines Spiralfadens besteht.

3. Vorrichtung nach einem der Ansprüche 1-2, wobei
mindestens ein Temperatursensor (123) zwischen dem Ballon (11) und der Elektrode (12) angeordnet ist, und
der Temperatursensor (123) über den mindestens einen Temperaturdraht (122), der in der Katheterkomponente (2) angeordnet ist, mit dem Hochfrequenz-Ablationsinstrument (4) verbunden ist.

4. Vorrichtung nach einem der Ansprüche 1-3, wobei,
eine Spitze (13) an einem vorderen Ende der Hochfrequenzkomponente (1) angeordnet ist und die Spitze (13) eine konische oder kugelförmige Struktur mit einem Durchmesser aufweist, der kleiner oder gleich dem Außendurchmesser der Katheterkomponente (2) ist.

5. Vorrichtung nach einem der Ansprüche 1-4, die ferner umfasst:
eine Hülle (5), die an einer Außenseite der Katheterkomponente (2) angebracht ist.

6. Vorrichtung nach einem der Ansprüche 1-5, wobei
die Elektrode (12) eine Kathode ist, und eine Anode (412) des Hochfrequenz-Ablationsinstruments (4) so konfiguriert ist, dass sie an einem menschlichen Körper angebracht werden kann.

## Revendications

1. Dispositif, comprenant
un composant de cathéter (2),
un composant radiofréquence (1), disposé sur une extrémité du composant de cathéter (2) s'étendant dans un corps humain, et
un instrument d'ablation par radiofréquence (4), disposé à l'autre extrémité du composant cathéter (2) et connecté au composant radiofréquence (1),
le composant radiofréquence (1) comprend un ballon (11) qui se dilate par pression de charge ou se rétracte par pression de décharge, et une électrode (12) disposée de manière fixe sur le ballon (11) ; et
un dispositif de pression de charge et de décharge est disposé à l'autre extrémité du composant de cathéter (2) et communique avec le ballon (11), l'électrode (12) est connectée à l'instrument d'ablation par radiofréquence (4) par l'intermédiaire d'un fil d'électrode (121) ;
dans lequel
le ballon (11) est cylindrique après avoir été expansé, ou une partie médiane du ballon (11) après avoir été expansée présente des saillies faisant saillie vers l'extérieur, de préférence, les saillies sont disposées autour d'un cercle de la ligne médiane du ballon (11) ou configurées en un angle défini, et une paroi latérale de la saillie à l'opposé du ballon (11) est parallèle à la ligne médiane du ballon (11) ;
la partie médiane du ballon (11), après avoir été expansée, présente une rainure évidée vers l'intérieur, et l'électrode (12) est fixée dans la rainure ;
la rainure est disposée autour d'un cercle de la ligne médiane du ballon (11) ou configurée selon un angle défini,
le composant de cathéter (2) comprend un tube externe (21) et un tube interne à cavités multiples (22) disposé dans le tube externe, et
le tube interne à cavités multiples (22) comporte une pluralité de canaux séparés, et la pluralité de canaux comprennent :
au moins un canal de fil (221) pour le placement d'un fil,
au moins un canal de chargement sous pression (222) pour l'entrée de liquide ou de gaz, et
un canal de filament de guidage (23) pour l'introduction du filament de guidage (7),
une extrémité du canal de chargement sous pression (222) qui s'étend jusqu'au ballon (11) est dotée d'un trou traversant sous pression (223) qui communique avec le ballon (11), le canal de chargement sous pression (222) et le ballon (11) sont reliés pour former une chambre étanche,
le tube interne à cavités multiples (22) est relié au ballon (11), et au moins un trou traversant pour fil (225) est disposé à proximité d'une partie de connexion du tube interne à cavités multiples (22) et du ballon (11) et utilisé pour faire communiquer le canal de fil (221) et un côté externe du tube interne à cavités multiples (22), et
un fil d'électrode (121) et au moins un fil de température (122) sont respectivement connectés à l'électrode correspondante (12) et à au moins un capteur de température (123) du composant radiofréquence (1) après avoir traversé le tube interne à cavités multiples (22) depuis le trou traversant pour fil (225),
l'autre extrémité du composant de cathéter (2) est dotée d'une valve à quatre voies (3), dans laquelle
une première ouverture de valve est configurée pour être reliée à une pompe à pression (6) par l'intermédiaire d'un cône Luer (32) prévu sur celle-ci,
une seconde ouverture de valve est connectée à l'instrument d'ablation par radiofréquence (4) par l'intermédiaire d'un connecteur électrique (31) prévu sur celle-ci, et
une troisième ouverture de valve (33) est configurée pour être utilisée pour faire passer le filament de guidage (7),
une structure de blocage (224) est disposée aux extrémités du canal de chargement sous pression (222) et du canal de fil (221), et la structure de blocage (224) scelle le canal de chargement sous pression (222) et le canal de fil (221),
un espace entre le trou traversant pour fil (225) et le fil est rempli de colle isolante.

2. Dispositif selon la revendication 1, dans lequel
l'électrode (12) est enroulée autour d'un côté externe du ballon (11) et a une forme sélectionnée dans le groupe constitué d'une forme de filament ou d'une forme de filament en spirale.

3. Dispositif selon l'une quelconque des revendications 1 et 2, dans lequel
l'au moins un capteur de température (123) est disposé entre le ballon (11) et l'électrode (12), et
le capteur de température (123) est connecté à l'instrument d'ablation par radiofréquence (4) à travers l'au moins un fil de température (122) disposé dans le composant de cathéter (2).

4. Dispositif selon l'une quelconque des revendications 1 à 3, dans lequel
une pointe (13) est en outre disposée au niveau d'une extrémité avant du composant radiofréquence (1), et la pointe (13) a une structure conique ou sphérique ayant un diamètre inférieur ou égal à un diamètre extérieur du composant de cathéter (2).

5. Dispositif selon l'une quelconque des revendications 1 à 4, comprenant en outre
une gaine (5) gainée sur un côté externe du composant de cathéter (2).

6. Dispositif selon l'une quelconque des revendications 1 à 5, dans lequel
l'électrode (12) est une cathode, et une anode (412) de l'instrument d'ablation par radiofréquence (4) est configurée pour être fixée à un corps humain.
